(19) Europäisches Patentamt
European Patent Office
Office européen des brevets

(11) Publication number: 0 295 815 A2

# (12) EUROPEAN PATENT APPLICATION

(21) Application number: 88305087.4

(51) Int. Cl.4: C07D 241/44 , B01J 27/00

(22) Date of filing: 03.06.88

(30) Priority: 19.06.87 US 65037

(43) Date of publication of application: 21.12.88 Bulletin 88/51

(84) Designated Contracting States: AT BE CH DE ES FR GB IT LI NL SE

(71) Applicant: UNIROYAL CHEMICAL COMPANY, Inc.
World Headquarters
Middlebury Connecticut 06749(US)

(72) Inventor: Malz, Russell E., Jr.
261 Wedgewood Drive Naugatuck
New Haven Connecticut 06770(US)

(74) Representative: Harrison, Michael Robert et al
Urquhart-Dykes & Lord 91 Wimpole Street
London W1M 8AH(GB)

## (54) Process for production of 2-quinoxalines.

(57) Certain 2-quinoxalinol-4-oxides are selectively reduced to form 2-quinoxalinols employing hydrogen as a reducing agent by reacting such 2-quinoxalinol-4-oxides with a catalyst selected from the group consisting of platinum, rhodium, ruthenium and nickel at between about 20° and about 150° C and at between about 6 and about 60 atmospheres.

EP 0 295 815 A2

# PROCESS FOR THE PRODUCTION OF 2-QUINOXALINES

## Field of the Invention

This invention is directed to an improved process for the production of certain 2-quinoxalinols by the selective reduction of 2-quinoxalinol-4-oxides employing a catalyst selected from the group consisting of platinum, rhodium, ruthenium and nickel in the presence of an aqueous hydroxide at between about 20° and about 150° C and at between about 6 and about 60 atmospheres, employing hydrogen as the reducing agent.

## Background of the Invention

2-quinoxalinol compounds, such as 6-chloro-2-hydroxyquinoxaline, are well known intermediates for the production of pharmaceutically and agriculturally effective chemicals. These compounds are generally prepared by the selective reduction of 2-quinoxalinol-4-oxides. Selective reduction is necessary so that excessive reduction, resulting in the formation of compounds such as 3,4-dihydro-2-quinoxalinol, can be avoided. This is particularly a problem with halogenated 2-quinoxalinol compounds wherein excessive hydrogenation will eliminate the halogen substituent.

Among the known processes for selectively reducing 2-quinoxalinol-4-oxides to 2-quinoxalinols is that described in United States Patent No. 4,636,562 to Davis. This patent discloses a process for preparing 2-chloro-6-haloquinoxaline compounds from the corresponding 4-halo-2-nitroaniline, which process includes a step involving the reduction of 6-chloro-2-hydroxyquinoline-4-oxide to 6-chloro-2-hydroxyquinoline employing hydrogen as a reducing agent in the presence of an aqueous alkali metal hydroxide solvent and a transition element metal hydrogenation catalyst, preferably Raney nickel. However, this Davis process is also not desirably commercially employed because Davis indicates that hydrogen pressures of 1-4 atmospheres are effective with pressures of 1-2 atmospheres being preferred. It is well known to those of ordinary skill in the art that processes involving gaseous hydrogen at such low pressures are inherently dangerous because there is a risk of air leaking into the system. If hydrogen and oxygen contact the hydrogenation catalyst together, water is formed in an explosive manner.

Somewhat similarly, Sakata et al, "The Facile One Pot Synthesis of 6-Substituted 2(1H)-quinoxalinones", Heterocycles, Vol. 23, No. 1 (1985), disclose a process for the reduction of 6-chloro-2-quinoxalinol-4-oxide to 6-chloro-2-quinoxalinol employing hydrogen as a reducing agent and palladium as a catalyst. However, Sakata et al indicate such process has the drawback that an undesirable amount of overly reduced by-product is also formed.

United States Patent No. 4,620,003 to Ishikura discloses a process for the reduction of 2-quinoxalinol-4-oxide compounds to 2-quinoxinol compounds, which process involves reacting a 2-quinoxalinol-4-oxide with hydrazine in the presence of (i) a Raney catalyst (especially Raney nickel or sulfided Raney nickel) and (ii) an alkali metal hydroxide, an alkaline earth metal hydroxide, or ammonium hydroxide. While this process reduces the 2-quinoxalinol-4-oxides in desirable efficiencies, a major drawback in the commercial use of the Ishikura process is the relatively high cost of hydrazine relative to other potential reducing agents, in particular hydrogen.

Consequently, it would be desirable to possess a process for the selective reduction of 2-quinoxalinol-4-oxides to 2-quinoxalinols safely and efficiently employing hydrogen as a reducing agent.

Accordingly, it is an object of this invention to provide a method of selectively reducing 2-quinoxalinol-4-oxides to 2-quinoxalinols employing hydrogen as a reducing agent.

It is a further object of this invention to provide a high pressure method of selectively reducing 2-quinoxalinol-4-oxides to 2-quinoxalinols employing hydrogen as a reducing agent such that the danger of air leaking into the system is minimized.

The above objects and additional objects will become more fully apparent from the following description and accompanying examples.

## Description of the Invention

The present invention is directed to a process for preparing a 2-quinoxalinol compound of the formula:

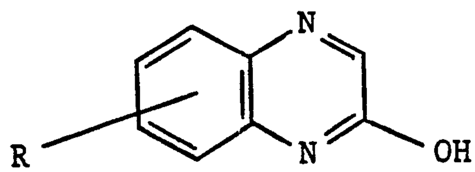

wherein R is hydrogen, halogen or trihalomethyl; which process comprises reducing a 2-quinoxalinol-4-oxide compound of the formula:

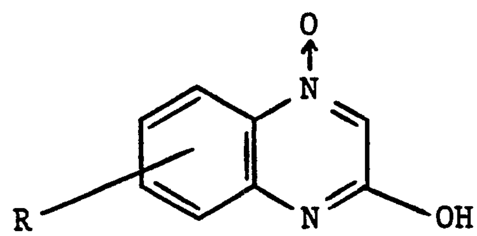

wherein R is as defined above, with hydrogen in the presence of a catalytically effective amount of at least one catalyst selected from the group consisting of platinum, rhodium, ruthenium and nickel at between about 20° and about 150° C and at between about 6 and about 60 atmospheres.

The 2-quinoxalinol-4-oxide compounds employed as starting materials in the process of this invention are of the formula:

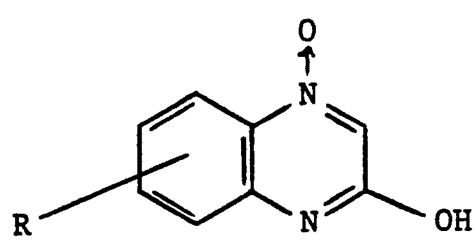

wherein R is hydrogen, halogen or trihalomethyl. Preferably, R is hydrogen, chlorine, fluorine, trichloromethyl or trifluoromethyl. A particularly preferred starting material is 6-chloro-2-hydroxyquinoxaline-4-oxide, which is subsequently reduced to 6-chloro-2-hydroxyquinoxaline.

These starting materials may be in isolated form or may be present as a reaction product. Thus, for example, 6-chloro-2-hydroxyquinoxaline-4-oxide may be present as the product of the reaction of 4-chloro-2-nitroacetoacetanilide with a hydroxide (such as sodium hydroxide or potassium hydroxide).

The process of this invention is catalyzed by a hydrogenation catalyst selected from the groups consisting of platinum, rhodium, ruthenium and nickel. Such catalysts are typically employed in amounts of about 0.01 to about 100 parts by weight per 100 parts by weight of 2-quinoxalinol-4-oxide.

When the starting material employed in the process of this invnetion is the reaction product of a nitroacetoanilide with a hydroxide, the reaction of this invention is conducted in a solution containing at least one compound selected from the group consisting of alkali metal hydroxides, alkaline earth metal hydroxides and ammonium hydroxide. Water is preferred as a reaction solvent but organic solvents (such as alcohols or hydrocarbons) or solvent mixtures of water with an organic solvent may also be employed.

The process of this invention is carried out at pressures between about 6 and about 60 atmospheres, with pressures of between about 8 and about 15 atmospheres being preferably employed. Reaction temperature may vary widely, with temperatures typically ranging between about 20° and about 150° C, and preferably ranging between about 30° and about 120° C.

The reaction temperatures and pressures which are most preferably employed will vary in accordance with a number of factors including the particular catalyst selected; the starting material employed; the catalyst concentration; and the like. As is apparent to one skilled in the art, reaction conditions which are too severe may reduce the selectivity of the reaction and produce products which may be too greatly reduced. Conversely, reaction conditions which are too mild will reduce the activity of the catalyst. Thus, for example, ruthenium under certain reaction conditions (i.e., a pressure of 200 psig and a concentration of 0.4 pph by weight of starting material) require a higher activation temperature (of above 60 °C). However, one of ordinary skill in the art may readily determine the optimum reaction conditions for a particular set of reactants by routine experimentation.

By employing methods well known to those skilled in the hydrogenation art, the process of this invention may be employed in a continuous or in a batch manner.

Because the process of this invention employs hydrogen as a reducing agent, it is greatly more economical than prior art processes employing relatively expensive chemicals (such as hydrazine) as reducing agents. Moreover, because the process of this invention is conducted at relatively high pressures, the danger of an explosion occurring due to the leaking in of air is greatly reduced.

## Examples

The following Examples are intended to further illustrate the invention and are not intended to limit the scope of the invention in any manner whatsoever.

## Examples 1-5 and Comparative Experiment A

To a 300 ml autoclave equipped with an agitator was added a stock solution containing 0.15 mole of 6-chloro-2-hydroxyquinoxaline-4-oxide (which had been produced by the reaction of 482.4 grams of 4-chloronitroacetoacetanilide with 813 grams of 89 potassium potassium hydroxide in 3200 grams of water) in a 17.6 weight percent aqueous potassium hydroxide solution. The amount and type of catalyst indicated in Table I below was added and the autoclave sealed. In this regard, it is to be noted that, with the exception of Example 4 (which employed Raney Nickel), all catalysts were 5 weight percent on carbon. The autoclave was purged, first with nitrogen and subsequently with hydrogen. Hydrogen was added until the reaction pressure was 200 psig. The autoclave was heated to 60 ° with agitation for the indicated reaction time, and was then cooled and depressurized. (Note: Example 5 was conducted at 120 °C and 200 psig; Comparative Experiment A was conducted at 30 °C and at 450-700 psig). The reaction product was removed with water, filtered through celite (to remove catalyst), and dried. The reaction product was analyzed, and the yield (based upon the complete cyclization, reduction, neutralization, filtration and drying steps) calculated. The results of such analyses are present in Table I.

TABLE I

| Reduction of 6-Chloro-2-Hydroxyquinoline-4-oxide to 6-Chloro-2-Hydroxyquinoline Employing $H_2$ as the Reducing Agent | | | | |
|---|---|---|---|---|
| Example or Comparative Experiment | Catalyst Type | Catalyst Amount(g) | Reaction Time (hours) | Percent Yield* |
| 1 | Pt | 0.25 | 5.5 | 68 |
| 2 | Rh | 0.40 | 3.0 | 45 |
| 3 | Rh | 0.08 | 1.5 | 70 |
| 4 | Ni | 6.2 | 0.3 | 76 |
| 5 | Ru | 1.0 | 5.2 | 75 |
| A | Pd | 1.0 | 0.08 | <0.5 |

* Based upon molar conversion of 4-chloronitroacetanilide into 6-chloro-2-hydroxyquinoline.

The above data indicate the admirable yields of selectively reduced product produced employing the process of this invention. These yields are unexpected in view of the lack of selectivity exhibited by a very closely related catalyst, palladium.

## Claims

1. A process for preparing a 2-quinoxalinol compound of the formula:

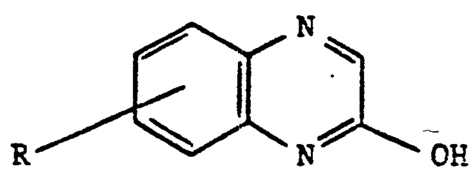

wherein R is hydrogen, halogen or trihalomethyl, characterised in that the process comprises reducing a 2-quinoxalinol-4-oxide compound of the formula:

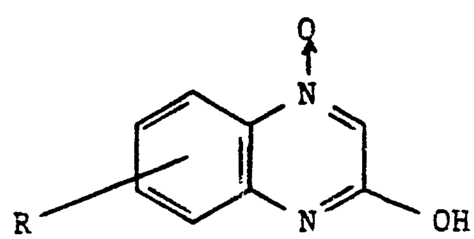

wherein R is as defined above, with hydrogen in the presence of a catalytically effective amount of at least one catalyst selected from platinum, rhodium, ruthenium and nickel at between about 20° and about 150°C and at between about 6 and 60 atmospheres.

2. A process according to claim 1 characterised in that said 2-quinoxalinol-4-oxide is in a solution further comprising at least one compound selected from alkali metal hydroxides, alkaline earth metal hydroxides and ammonium hydroxide.

3. A process according to any of the preceding claims characterised in that R is selected from hydrogen, chlorine, fluorine, trichloromethyl and trifluoromethyl.

4. A process according to any of the preceding claims characterised in that the starting material is 6-chloro-2-hydroxyquinoline-4-oxide.

5. A process according to any of the preceding claims characterised in that the reduction takes place at between about 30° and about 120° C.

6. A process according to any of the preceding claims characterised in that the reduction takes place at between about 8 and about 15 atmospheres.

7. A process for preparing 6-chloro-2-hydroxyquinoline from 6-chloro-2-hydroxyquinoline-4-oxide which process reacting 6-chloro-2-hydroxyquinoline-4-oxide with hydrogen in the presence of (a) a catalytically effective amount of at least one member selected from platinum, rhodium, ruthenium and nickel and (b) an aqueous solution of an alkali metal hydroxide at between about 20° and about 150° C and at between about 6 and about 60 atmospheres.

8. A process according to claim 7 characterised in that such reaction occurs at between about 30° and about 120° C and at between about 8 and about 15 atmospheres.